(19) Europäisches Patentamt
European Patent Office
Office européen des brevets

(11) Veröffentlichungsnummer: **0 056 560**
**A1**

# (12) EUROPÄISCHE PATENTANMELDUNG

(21) Anmeldenummer: **81810013.3**

(22) Anmeldetag: **19.01.81**

(51) Int. Cl.³: **A 61 K 45/06**
A 61 K 37/02, A 61 K 31/70
A 61 K 31/71, A 23 K 1/16
A 23 K 1/17
//(A61K37/02, 31/71), (A61K37/02, 31/43), (A61K37/02, 31/65), (A61K31/71, 31/70)

(43) Veröffentlichungstag der Anmeldung:
**28.07.82 Patentblatt 82/30**

(84) Benannte Vertragsstaaten:
**BE CH DE FR GB IT LI LU NL SE**

(71) Anmelder: **CIBA-GEIGY AG**
**Patentabteilung Postfach**
**CH-4002 Basel(CH)**

(72) Erfinder: **Tarcsay, Lajos, Dr.**
**Muttenzerstrasse 25**
**D-7889 Grenzach-Wyhlen(DE)**

(72) Erfinder: **Baschang, Gerhard, Dr.**
**Bückenweg 7**
**CH-4126 Bettingen(CH)**

(72) Erfinder: **Hartmann, Albert, Dr.**
**Steingasse 21A**
**D-7889 Grenzach(DE)**

(72) Erfinder: **Stanek, Jaroslav, Dr.**
**Florastrasse 6**
**CH-4127 Birsfelden(CH)**

## (54) Antibiotische Präparate mit gesteigerter Wirksamkeit, Verfahren zu deren Herstellung und Verfahren zur Steigerung der antibiotischen Wirkung von Antibiotika.

(57) Die Erfindung betrifft pharmazeutische Präparate, die ein Antibiotikum und ein Muramylpeptid der Formel I oder ein Salz desselben enthalten, Verfahren zu deren Herstellung und ein Verfahren zur Steigerung der antibiotischen Wirksamkeit von Antibiotika.

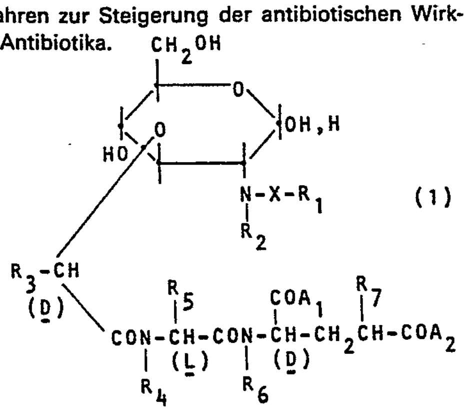

(1)

In der Formel I bedeuten X Carbonyl oder Carbonyloxy, $R_1$ gegebenenfalls substituiertes Alkyl oder Aryl, $R_2$, $R_3$, $R_4$ und $R_6$ Wasserstoff oder Niederalkyl, $R_5$ Wasserstoff, gegebenenfalls substituiertes Niederalkyl, Cycloalkyl, Aryl oder stickstoffhaltiges Heterocyclyl, oder $R_4$ und $R_5$ zusammen auch $C_3$-$C_4$-Alkylen, $R_7$ Wasserstoff oder freies, verestertes oder amidiertes Carboxyl, einer der Reste $A_1$ und $A_2$ einen Rest der Formel II und der andere der Reste $A_1$ und $A_2$ gegebenenfalls substituiertes oder funktionell abgewandeltes Hydroxy oder Amino.

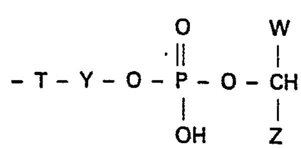

(II)

In der Formel II bedeuten T NH oder O, Y eine gegebenenfalls substituierte Alkylengruppe, die auch durch Oxy- oder Iminocarbonyl unterbrochen sein kann, W Wasserstoff und Z eine gegebenenfalls veresterte oder verätherte 1,2-Dihydroxyäthyl- oder 2-Hydroxyäthylgruppe, oder W und Z eine gegebenenfalls veresterte oder verätherte Hydroxymethylgruppe.

EP 0 056 560 A1

CIBA-GEIGY AG
Basel (Schweiz)

4-13194

# Antibiotische Präparate mit gesteigerter Wirksamkeit, Verfahren zu deren Herstellung und Verfahren zur Steigerung der antibiotischen Wirkung von Antibiotika

Die vorliegende Erfindung betrifft antibiotische Präparate mit verstärkter Wirksamkeit, Verfahren zu deren Herstellung und ein Verfahren zur Steigerung der antibiotischen Wirksamkeit von Antibiotika.

Die Erfindung betrifft insbesondere Antibiotikapräparate, die mindestens ein Antibiotikum und mindestens ein Muramylpeptid der Formel (I) oder ein Salz desselben enthalten,

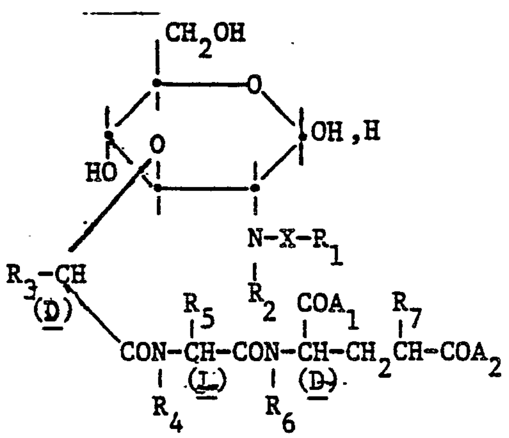

(I)

worin X Carbonyl oder Carbonyloxy, $R_1$ gegebenenfalls substituiertes Alkyl oder Aryl, $R_2$, $R_4$ und $R_6$ Wasserstoff oder Niederalkyl, $R_3$ Wasserstoff oder Niederalkyl, $R_5$ Wasserstoff, Niederalkyl, freies oder funktionell abgewandeltes Hydroxyniederalkyl, freies oder funktionell abgewandeltes Mercaptoniederalkyl, gegebenenfalls substituiertes Aminoniederalkyl, Cycloalkyl, Cycloalkylniederalkyl, gegebenenfalls substituiertes Aryl oder Aralkyl, stickstoffhaltiges

Heterocyclyl oder Heterocyclylniederalkyl, oder $R_4$ und $R_5$ zusammen auch Alkylen mit 3-4 Kohlenstoffatomen, $R_7$ Wasserstoff oder gegebenenfalls verestertes oder amidiertes Carboxyl und einer der Reste $A_1$ und $A_2$ einen Rest der Formel

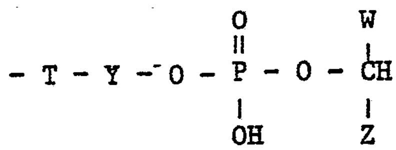

(II)

bedeutet, worin T für NH oder O steht, Y eine gegebenenfalls substituierte Alkylengruppe, die auch durch ein oder zwei Oxycarbonyl und/oder Iminocarbonyl unterbrochen sein kann, W Wasserstoff und Z eine 1,2-Dihydroxyäthyl- oder 2-Hydroxyäthylgruppe darstellen, in denen mindestens eine Hydroxygruppe mit einer gegebenenfalls ungesättigten, langkettigen aliphatischen Carbonsäure verestert oder mit einem gegebenenfalls ungesättigten, langkettigen aliphatischen Alkohol veräthert ist, oder W und Z je eine mit einer gegebenenfalls ungesättigten langkettigen aliphatischen Carbonsäure veresterte oder mit einem gegebenenfalls ungesättigten langkettigen aliphatischen Alkohol verätherte Hydroxymethylgruppe darstellen, und der andere der Reste $A_1$ und $A_2$ freies oder veräthertes Hydroxy, freies oder alkyliertes Amino, Niederalkylamino oder Aminocarbonylniederalkylamino ist und ihre Salze.

Alkyl ist insbesondere geradkettiges oder verzweigtes, in beliebiger Stellung gebundenes Alkyl mit bis zu 18 Kohlenstoffatomen, in erster Linie jedoch Niederalkyl.

Als Substituenten der gegebenenfalls substituierten Alkylgruppen kommen in erster Linie freie oder funktionell abgewandelte Hydroxy- oder Mercaptogruppen, wie verätherte oder veresterte Hydroxy- oder Mercaptogruppen, z.B. Niederalkoxy oder Niederalkylmercaptogruppen, oder Halogenatome oder freie oder funktionell abgewandelte Carboxyl-, wie Niederalkoxycarbonyl- oder Carbamoylgruppen in Frage. Dabei kann

der substituierte Alkylrest, wie Niederalkylrest, einen, zwei oder mehrere gleiche oder verschiedene Substituenten, insbesondere freie Hydroxylgruppen oder Halogenatome, tragen.

Arylreste sind insbesondere monocyclische sowie bicyclische Arylreste, in erster Linie Phenyl, aber auch Naphthyl. Sie können gegebenenfalls, z.B. durch Niederalkylgruppen, freies, verestertes oder veräthertes Hydroxy, z.B. Niederalkoxy oder Niederalkylendioxy oder Halogenatome, und/oder Trifluormethylgruppen, mono-, di- oder polysubstituiert sein.

Aralkyl ist insbesondere Arylniederalkyl, worin Aryl die oben gegebene Bedeutung hat. In erster Linie steht Arylniederalkyl für Benzyl oder Phenyläthyl, worin der Phenylkern mono-, di- oder polysubstituiert sein kann.

Gegebenenfalls substituierte Aralkylreste sind insbesondere solche Reste, die im aromatischen Kern gegebenenfalls mono-, di- oder polysubstituiert sind, z.B. durch Niederalkyl, freie, verätherte oder veresterte Hydroxy- oder Mercaptogruppen, z.B. Niederalkoxy- oder Niederalkylendioxy- sowie Niederalkylmercapto- oder Trifluormethylgruppen und/oder Halogenatome.

Cycloalkyl ist insbesondere Cycloalkyl mit 5 bis 6 Kohlenstoffatomen, wie Cyclopentyl oder Cyclohexyl und Cycloalkylniederalkyl insbesondere ein solcher Rest, worin der Cycloalkylteil 5 bis 6 Kohlenstoffatome besitzt und der Niederalkylteil in erster Linie Methyl oder Aethyl darstellt.

Stickstoffhaltiges Heterocyclyl ist insbesondere der Rest einer 5- oder 6-gliedrigen, ein oder zwei Stickstoffatome im Ring enthaltenden heterocyclischen Verbindung. Er kann ungesättigt oder auch gesättigt

sein und z.B. einen ankondensierten Phenylrest enthalten. Als solche seien z.B. die Pyrrolyl-, Indolyl-, Pyridyl- oder Imidazolylreste genannt.

Im stickstoffhaltigen Heterocyclylniederalkyl hat der Heterocyclylrest die obengenannte Bedeutung und der Niederalkylrest ist in erster Linie Methyl oder Aethyl.

Der Alkylenrest, der von den Resten $R_4$ und $R_5$ gebildet sein kann, ist vorzugsweise unsubstituiert und in erster Linie der Trimethylenrest.

Eine gegebenenfalls veresterte oder amidierte Carboxylgruppe ist in erster Linie die Carboxylgruppe selbst oder eine mit einem Niederalkanol veresterte Carboxylgruppe oder auch die Carbamoylgruppe, die am Stickstoffatom unsubstituiert oder mit Alkyl, insbesondere Niederalkyl, Aryl, in erster Linie Phenyl, oder Aralkyl, wie Benzyl, mono- oder disubstituiert ist. Die Carbamoylgruppe kann aber auch einen Alkylen-, wie den Tetra- oder Pentamethylenrest tragen.

Als gegebenenfalls funktionell abgewandelte Hydroxy- oder Mercaptogruppen sind insbesondere verätherte oder veresterte Hydroxy- oder Mercaptogruppen zu nennen, wie Niederalkoxy, Niederacyloxy, z.B. Niederalkanoyloxy, oder Halogenatome, Niederalkylmercapto oder Niederacylmercapto, z.B. Niederalkanoylmercapto.

Als funktionell abgewandeltes Aminoniederalkyl sind insbesondere Mono- oder Diniederalkylaminoniederalkyl oder acyliertes Aminoniederalkyl, wie Methylamino-, Aethylamino-, Dimethylamino-, Diäthylamino- und Alkanoylaminoniederalkyl, z.B. Niederalkanoylaminoniederalkyl, zu nennen.
Aminocarbonylniederalkylamino ist in erster Linie 1-Aminocarbonylniederalkylamino, z.B. Glycylamino, Alanylamino, Valylamino oder

Isoleucylamino.

Der Alkylenrest Y enthält bis zu 20 C-Atome und ist insbesondere ein Niederalkylenrest, vorzugsweise mit 2 oder 3 Kohlenstoffatomen. Der Alkylenrest Y kann aber auch ein durch einen Rest wie Oxycarbonyl oder N-$R^a$-Iminocarbonyl unterbrochener Niederalkylenrest sein, und stellt dann insbesondere einen Rest einer der Formeln

$$-Y_1-COO-Y_2- \qquad \text{(IIIa)}$$

$$-Y_1-OOC-Y_2- \qquad \text{(IIIb)}$$

$$-Y_1-\underset{R^a}{\underset{|}{CON}}-Y_2- \qquad \text{(IIIc)}$$

bzw. $$-Y_1-\underset{R^a}{\underset{|}{NOC}}-Y_2 \qquad \text{(IIId)}$$

dar, worin einer der Reste $Y_1$ und $Y_2$ einen gegebenenfalls substituierten Niederalkylenrest und der andere einen gegebenenfalls substituierten Niederalkylenrest, der auch durch Oxycarbonyl oder N-$R^a$-Iminocarbonyl unterbrochen sein kann, wobei $Y_1$ und $Y_2$ zusammen mehr als zwei Kohlenstoffatome aufweisen, und $R^a$ Wasserstoff oder Niederalkyl bedeuten. Als Substituenten der Reste $Y_1$ und $Y_2$ sollen insbesondere freies oder funktionell abgewandeltes Hydroxy oder Hydroxyniederalkyl, freies oder funktionell abgewandeltes Mercapto oder Mercaptoniederalkyl, freies, mono- oder diniederalkyliertes oder acyliertes Aminoniederalkyl, Aminocarbonyl, Alkyl, Cycloalkyl mit 5 bis 6 Kohlenstoffatomen, Aryl oder Aralkyl genannt werden, wobei die Allgemeinbegriffe die oben angegebene Bedeutung haben können.

Eine langkettige aliphatische Carbonsäure ist insbesondere eine mit 10 bis 90 Kohlenstoffatomen, die auch 1 oder 2 Doppelbindungen aufweisen und gerade oder verzweigt sein kann. Bevorzugt sind solche mit bis zu 30, in erster Linie mit 16 bis 22 Kohlenstoffatomen oder natürliche oder synthetische Mycolsäuren.

Ein langkettiger aliphatischer Alkohol ist insbesondere ein Alkanol mit bis zu 30, in erster Linie mit 10-22 Kohlenstoffatomen, das auch eine oder zwei Doppelbindungen aufweisen und gerade oder verzweigt sein kann. Bevorzugt sind solche Alkanole, die 12-18 Kohlenstoffatome enthalten und deren Hydroxygruppe endständig ist.

Die im Zusammenhang mit der vorliegenden Beschreibung und den Patentansprüchen mit "Nieder" bezeichneten Reste und Verbindungen enthalten vorzugsweise bis und mit 7 und in erster Linie bis und mit 4 Kohlenstoffatome.

Vorstehend wie nachfolgend können die Allgemeinbegriffe folgende Bedeutung haben:

Niederalkyl ist z.B. n-Propyl, n-Butyl, Isobutyl, sek.-Butyl oder tert.-Butyl, ferner n-Pentyl, n-Hexyl, Isohexyl oder n-Heptyl und in erster Linie Methyl oder Aethyl. In Aryl-, Cycloalkyl- oder Heterocyclylniederalkylresten ist der Niederalkylrest insbesondere Methyl oder Aethyl, wobei der Aryl-, Cycloalkyl- oder Heterocyclylrest die obengenannte Bedeutung besitzt.

Niederalkoxy ist z.B. n-Propoxy, n-Butoxy, Isobutoxy, sek.-Butoxy oder tert.-Butoxy und in erster Linie Methoxy oder Aethoxy.

Niederalkylmercapto ist z.B. n-Propyl-, n-Butyl-, Isobutyl-, sek.-Butyl- oder tert.-Butylmercapto und in erster Linie Methylmercapto oder Aethylmercapto.

Niederalkylendioxy ist insbesondere Methylendioxy, Aethylen- oder Propylendioxy.

Halogen steht für Fluor oder Brom, vorzugsweise jedoch für Chlor.

Niederalkanoyl ist insbesondere Propionyl oder Butyryl, in erster Linie jedoch Acetyl.

Synthetische Mycolsäuren sind insbesondere α-Alkyl-β-hydroxyalkancarbonsäuren, worin der Alkylrest in α-Stellung 1-20, in erster Linie 1-14 und die Alkancarbonsäure 20-80, insbesondere 30-34 Kohlenstoffatome enthalten. Sie können auch weitere Hydroxylgruppen, sowie Oxo-, Methylen- oder Aethylengruppen enthalten.

Natürliche Mycolsäuren sind insbesondere solche, wie sie aus lebenden Organismen, wie Bakterien, z.B. Mycobakterien, isoliert werden können.

Die Verbindungen der Formel I können in Form von Isomerengemischen oder reinen Isomeren vorliegen. Vorzugsweise liegt der mit dem Sauerstoffatom verknüpfte Rest der Formel $-CH(R_3)-(C{=}O)-$, falls $R_3$ für Niederalkyl steht, in optisch aktiver Form vor und hat insbesondere die D-Form, während der Rest der Aminosäure der Formel $-N(R_4)-CH(R_5)-C({=}O)-$, falls $R_5$ von Wasserstoff verschieden ist, ebenfalls vorzugsweise in optisch aktiver, in erster Linie in der L-Form vorliegt, und der terminale α-Amino-glutarsäurerest vorzugsweise in optisch aktiver, insbesondere in der D-Form, vorliegt. Ferner kann die 1-Hydroxygruppe die α- oder die β-Konfiguration haben; die neuen Verbindungen der Formel I können jedoch auch als Gemisch der 1α- und 1β-Isomeren vorliegen.

In den Verbindungen der Formel I kann das über ein Sauerstoffatom an Phosphor gebundene Proton leicht mit Basen abgespalten werden. Ueblicherweise liegen die Verbindungen der Formel I in Form eines Gemisches der freien Verbindungen und ihrer Salze vor. So liegen etwa 40-55% der in den Beispielen beschriebenen Muramylpeptide der Formel I als Natriumsalze vor. Diese Salze gehören als Kombinationspartner mit zum Gegenstand der Erfindung.

Die Erfindung betrifft generell auch die Salze der Verbindungen der Formel I mit irgendwelchen anderen salzbildenden Gruppen, z.B. freien Carboxylgruppen, in erster Linie pharmazeutisch verwendbare, nichttoxische Salze, z.B. Metall- oder Ammoniumsalze.

Verbindungen der Formel I mit Aminogruppen, beispielsweise im Rest $R_5$, können als innere Salze (Zwitterionen) oder als Säureadditionssalze vorliegen. Zur Addition geeignet sind vor allem schwache und pharmazeutisch verwendbare Säuren. Als Kombinationspartner kommen beliebige Antibiotika in Frage. Man kann einzelne Antibiotika wie auch Antibiotikagemische verwenden. Letztere enthalten bevorzugterweise nicht mehr als drei Verbindungen mit antibiotischer Wirkung. In erster Linie verwendet man Antibiotika aus der Gruppe der β-Lactamantibiotika, Aminoglycoside, Tetracycline, Macrolide, Lincomycine, Polyenantibiotika, Polypeptidantibiotika, Anthracycline, Chlor- oder Thiamphenicole, Cycloserine, Fusidinsäuren oder Rifamycine.

Als bevorzugte Antibiotika seien unter den β-Lactamen die Penicilline, Cephalosporine, Peneme, Nocardicine, Thienamycine und Clavulanverbindungen, genannt.

Penicillin-Antibiotika sind in erster Linie Amoxycillin, Ampicillin, Carbenicillin, Cloxacillin, Cyclacillin, Dicloxacillin, Mecillinam, Methicillin, Penicillin G, Penicillin V, Pivampicillin, Sulbenicillin, Azlocillin, Ticarcillin, Mezlocillin, Pivmecillinam oder 6-(4-endo-Azatricyclo[5.2.2.$0^{2,6}$]undec-8-enyl)-methylenaminopenicillansäure genannt.

Aus der Gruppe der Cephalosporine können z.B. Cefaclor, Cefazaflur, Cefazolin, Cefadroxil, Cefoxitin, Cefuroxim, Cephacetril, Cephalexin, Cephaloglycin, Cephaloridine, Cephalotin, Cefamandol, Cephanon, Cephapirin, Cefatrizin, Cephradin, Cefroxadin (7β-[D-2-amino-2-(1,4-cyclohexadienyl)-acetamido]-3-methoxy-3-cephem-4-carbonsäure = CGP 9000), Cefsulodin, Cefotaxim, Cefotiam, Ceftezol oder Cefazedon genannt werden.

Unter den Nocardicinen ist z.B. Nocardicin A und unter den Thienamycinen und Clavulansäuren z.B. Thienamycin bzw. Clavulansäure zu erwähnen.

Unter den Aminoglycosiden sind insbesondere Streptomycine, z.B. Streptomycin und Streptomycin A, Neomycine, z.B. Neomycin B, Tobramycine, z.B. Tobramycin oder Dibekacin, Kanamycine (z.B. Gemische von Kanamycin A, B und C), sowie Amicacine, Gentamycine (z.B. Gemische von Gentamycin A, $C_1$, $C_2$ oder $C_{1a}$) oder Sisomicine, wie Sisomicin oder Netilmicin, ferner Lividomycin, Ribocamycin und Paromomycin zu erwähnen.

Als Tetracycline sind insbesondere Tetracyclin, Doxycyclin, Chlortetracyclin, Oxytetracyclin oder Methacyclin zu nennen.

Als Macrolide sind z.B. Maridomycin, Spiramycine, wie Spiramycin I, II und III, Erythomycine, z.B. Erythromycin, Oleandomycine, z.B. Oleandomycin und Tetraacetyloleandomycin, und als Lincomycine z.B. Lincomycin und Clindamycin zu erwähnen.

Als Polyen-Antibiotika sind besonders Amphotericin B und dessen Methylester oder Nystalin zu nennen.

Als Polypeptid-Antibiotika können z.B. Colistin, Gramicidin S, Polymyxin B, Virginamycin, Tyrothricin, Viomycin oder Vancomycin besonders genannt werden.

Als Rifamycine kommen in erster Linie das Rifamycin S, Rifamycin SV oder Rifamycin B oder deren halbsynthetische Derivate, insbesondere

das Rifampicin, in Frage.

Die erfindungsgemässen Kombinationspräparate weisen pro Dosiseinheit die üblichen Mengen an Antibiotika auf, z.B. zwischen 50 und 1000 mg, in der Regel zwischen 100 und 500 mg. Die Muramylpeptidmenge richtet sich nach dem vorgesehenen Applikationsort. Sie ist für oral verabreichbare Präparate höher als für injizierbare. Oral applizierbare Präparate enthalten zwischen 1 mg bis zur Hälfte der Antibiotikamenge an Muralmylpeptid der Formel I, in der Regel zwischen 5 und 50 mg. Bei Verwendung von magensaftbeständigen Manteltabletten kann die Dosierung auch weniger als 1 mg (bis zu 0,01 mg) Muramylpeptid pro Tablette betragen. Injizierbare Präparate enthalten zwischen 10 µg und 50 mg, bevorzugt zwischen 100 µg und 10 mg Muramylpeptid. Diese Präparate können, insbesondere wenn sie für orale Gaben zu verwenden sind, ausserdem noch die üblichen Mengen an pharmakologischen Trägerstoffen, Streck- und/oder Verdünnungsmitteln enthalten. Insbesondere für injizierbare Präparate sind auch liposomale Applikationsformen geeignet.

Besonders hervorzuheben sind pharmazeutische oder veterinärmedizinische Präparate, sowie Futtermittel oder Futtermittelzusätze, die eine wirksame oder unterwirksame Dosis der genannten Antibiotika und zusätzlich ein Muramylpeptid der Formel I enthalten.

Die pharmazeutischen Präparate der vorliegenden Erfindung sind vorzugsweise Tabletten oder Gelatinekapseln, welche die Wirkstoffe zusammen mit Verdünnungsmitteln, z.B. Laktose, Dextrose, Saccharose, Mannit, Sorbit, Cellulose und/oder Glucose und Schmiermitteln, z.B. Kieselerde, Talk, Stearinsäure oder Salze davon, wie Magnesium- oder Calciumstearat, und/oder Polyäthylenglykol, enthalten. Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel , z.B. Stärken, Agar, Alginsäure oder ein Salz davon, wie Natriumalginat und/oder Brausemischungen, oder Adsorptionsmittel, Farbstoffe, Geschmackstoffe und Süssmittel. Injizierbare Präparate sind vorzugsweise isotonische wässrige Lösungen oder Suspensionen. Suppositorien, Salben oder Cremen sind in erster Linie Fettemulsionen oder -suspensionen. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1% bis etwa 75%, insbesondere von etwa 1% bis etwa 50%, der genannten Aktivstoffe.

Die oral applizierbaren Präparate der vorliegenden Erfindung können auch mit einem gegen Magensaft beständigen Ueberzug versehen sein.

Die hohe antibiotische Wirkung der neuen Präparate und des neuen Verfahrens kann durch "in vivo"-Versuche nachgewiesen werden, die an verschiedenen Tierarten, insbesondere an Säugetieren, wie Mäusen, durchgeführt werden. Dazu infiziert man sie mit einer letalen oder subletalen Dosis eines pathogenen Mikroorganismus und gibt dann das genannte neue Präparat bzw. die einzelnen Dosen an Muramylpeptid und Antibiotikum. Die Wirkung wird als $ED_{50}$ bestimmt, das ist diejenige Dosis, bei der 50% der Tiere überleben.

Es wurde nun überraschenderweise gefunden, dass die Infektion mit pathogenen Keimen, insbesondere der weniger beeinflussbaren gramnegativen Bakterien, wie z.B. Stämmen der Aerobakter, Brucella, Escherichia, Klebsiella, Malleomyces, Neisseria, Pasteurella, Proteus,

Pseudomonas, Shigella und Vibro, aber auch von gram-positiven Bakterien, wie von Aktinomycetes, Clostridia, Corynebakterien, Diplokokken, Mycobakterien oder Staphylokokken, oder von Pilzen, wie Candida Albicans, Cryptokokkus neoformans, Plastomyces dermatitides oder Hystoplasma capsulatum, in erhöhtem Masse gehemmt und bekämpft wird.

Die Erfindung betrifft auch ein Verfahren zur Steigerung der antibiotischen Aktivität von Antibiotika. Dieses Verfahren ist dadurch gekennzeichnet, dass man mindestens ein Antibiotikum, insbesondere ein solches wie bei den erfindungsgemässen Kombinationspräparaten beschrieben, mit mindestens einem der vorgenannten Muramylpeptide der Formel I zusammen verabreicht.

Im Verfahren der vorliegenden Erfindung verwendet man eine wirksame oder unterwirksame Dosis des Antibiotikums, je nach Art des letzteren z.B. von etwa 10 bis etwa 1000 mg, insbesondere von etwa 50 bis etwa 500 mg pro Einzeldosis.

Die Dosierung der Muramylpeptide der Formel I richtet sich nach dem Applikationsort und entspricht der für die pharmazeutischen Präparate genannten Dosierung. Dabei kann das Muramylpeptid-Derivat bis zu 24 Stunden vor oder nach, vorzugsweise jedoch etwa gleichzeitig mit dem Antibiotikum verabreicht werden.

Die Verabreichung der Antibiotika erfolgt auf dem üblichen Wege, wie subkutan, intravenös oder oral, während man die Muramylpeptide, insbesondere wenn sie getrennt von den Antibiotika verabreicht werden, meist subkutan verabfolgt.

Als Kombinationspartner kommen insbesondere Muramylpeptide der Formel I in Frage,

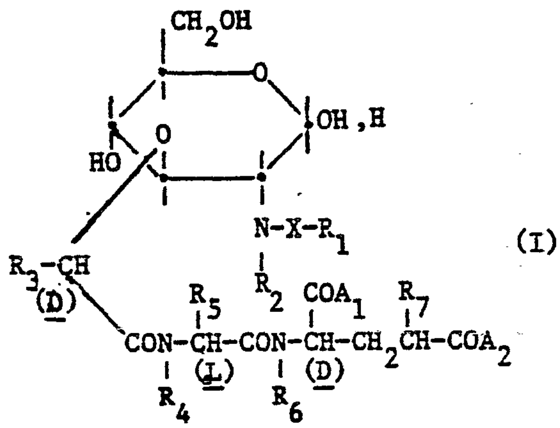

(I)

worin X Carbonyl, $R_1$ gegebenenfalls substituiertes Alkyl mit bis zu 18 C-Atomen oder Aryl mit bis zu 30 C-Atomen, $R_2$, $R_3$, $R_4$ und $R_6$ Wasserstoff oder Niederalkyl, $R_5$ Wasserstoff, gegebenenfalls durch Hydroxy, Niederalkoxy, Mercapto, Niederalkylmercapto, Amino, Niederalkylamino oder Halogen substituiertes Niederalkyl, Cycloalkyl oder Cycloalkylniederalkyl, worin der Cycloalkylrest 4-6 Kohlenstoffatome enthält, gegebenenfalls substituiertes Phenyl oder Phenylniederalkyl, ein oder zwei Azaatome enthaltendes Heterocyclyl oder Heterocyclylniederalkyl mit jeweils 5-6 Ringgliedern, oder $R_4$ und $R_5$ zusammen auch Alkylen mit 3-4 Kohlenstoffatomen, $R_7$ Wasserstoff und einer der Reste $A_1$ und $A_2$ einen Rest der Formel

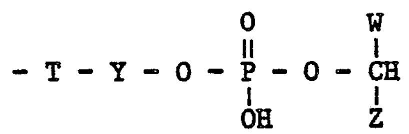

(II)

bedeuten, worin T für HN oder O steht, Y gegebenenfalls substituiertes Alkylen mit bis zu 20 C-Atomen, das auch durch Carbonyloxy oder Carbonylimino unterbrochen sein kann, W Wasserstoff und Z eine 1,2-Dihydroxyäthyl- oder 2-Hydroxyäthylgruppe darstellen, in denen mindestens eine Hydroxygruppe mit einer gegebenenfalls ungesättigten langkettigen aliphatischen Carbonsäure mit bis zu 30 C-Atomen oder mit einer Mycolsäure verestert oder mit einem gegebenenfalls ungesättigten langkettigen aliphatischen Alkohol mit bis zu 30 C-Atomen veräthert ist, oder W und Z je eine mit einer gegebenenfalls ungesättigten langkettigen aliphatischen Carbonsäure mit bis zu 30 C-Atomen oder mit einer Mycolsäure veresterte oder mit einem gegebenenfalls ungesättigten langkettigen aliphatischen Alkohol mit bis zu 30 C-Atomen verätherte Hydroxymethylgruppe darstellen, und der andere der Reste $A_1$ und $A_2$ freies oder veräthertes Hydroxy, Amino, Niederalkylamino oder Aminocarbonylniederalkylamino ist, und ihre Salze.

Bevorzugte Kombinationspartner sind Muramylpeptide der Formel I, worin X Carbonyl, $R_1$ gegebenenfalls durch Hydroxy, Niederalkoxy oder Halogen substituiertes Niederalkyl oder gegebenenfalls durch Hydroxy, Niederalkoxy, Niederalkyl oder Halogen substituiertes Phenyl, $R_2$, $R_4$ und $R_6$ Wasserstoff oder Methyl, $R_3$ Wasserstoff, Methyl oder Aethyl, $R_5$ Wasserstoff, gegebenenfalls durch Hydroxy, Niederalkoxy, Mercapto, Niederalkylmercapto oder Halogen substituiertes Niederalkyl mit 1-7 Kohlenstoffatomen, 4-Amino-butyl, Cycloalkyl oder Cycloalkylniederalkyl, worin der Cycloalkylrest 4-6 Kohlenstoffatome und der Niederalkylrest 1-3 Kohlenstoffatome enthält, gegebenenfalls durch Hydroxy, Niederalkoxy oder Halogen substituiertes Phenyl oder Phenylniederalkyl mit 1-3 Kohlenstoffatomen im Niederalkylrest, 4-Imidazolyl-methyl, 3-Indolyl-methyl oder $R_4$ und $R_5$ zusammen auch Alkylen mit 3-4 Kohlenstoffatomen, $R_7$ Wasserstoff und einer der Reste $A_1$ und $A_2$ einen Rest der Formel

$$-T-Y-O-\underset{\substack{|\\ OH}}{\overset{\substack{O\\ \|}}{P}}-O-\underset{\substack{|\\ Z}}{\overset{\substack{W\\ |}}{CH}} \qquad (II)$$

bedeutet, worin T für HN oder O steht, Y gegebenenfalls substituiertes Niederalkylen oder einen Rest der Formeln

$$-Y_1-COO-Y_2 \qquad (IIIa)$$

oder

$$-Y_1-CONH-Y_2 \qquad (IIIe)$$

bedeutet, worin $Y_1$ und $Y_2$ unabhängig voneinander je für Niederalkylen

mit 1-7 Kohlenstoffatomen stehen, das gegebenenfalls durch Hydroxy, Niederalkoxy, Mercapto, Methylthio, Phenyl, 4-Imidazolyl oder 3-Indolyl substituiert ist, W Wasserstoff, und Z eine 1,2-Dihydroxyäthyl- oder 2-Hydroxyäthylgruppe darstellen, in denen mindestens eine Hydroxygruppe mit einer gegebenenfalls ein- oder zweifach ungesättigten aliphatischen Carbonsäure mit 14-24 Kohlenstoffatomen oder mit einer natürlichen oder synthetischen Mycolsäure verestert oder mit einem gegebenenfalls ein- oder zweifach ungesättigten aliphatischen Alkohol mit 10-20 Kohlenstoffatomen veräthert ist,oder W und Z je eine mit einer gegebenenfalls ein- oder zweifach ungesättigten aliphatischen Carbonsäure mit 16-22 C-Atomen oder mit einer natürlichen oder synthetischen Mycolsäure veresterte oder mit einem gegebenenfalls ein- oder zweifach ungesättigten aliphatischen Alkohol mit 12-18 C-Atomen verätherte Hydroxymethylgruppe bedeuten, und der andere der Reste $A_1$ und $A_2$ Hydroxy, Niederalkoxy, Amino, Niederalkylamino oder Aminocarbonylniederalkylamino ist und ihre Salze.

Besonders bevorzugte Kombinationspartner sind Muramylpeptide der Formel I, worin X Carbonyl, $R_1$ Niederalkyl oder Phenyl, $R_2$, $R_6$ und $R_7$ Wasserstoff, $R_3$ und $R_4$ Wasserstoff oder Methyl, $R_5$ Wasserstoff, gegebenenfalls durch Phenyl substituiertes Niederalkyl mit 1-7 C-Atomen oder $R_4$ und $R_5$ zusammen auch Trimethylen bedeuten, worin $A_1$ für Hydroxy, Niederalkoxy, Amino, Niederalkylamino oder Aminocarbonylniederalkylamino steht und $A_2$ einen Rest der Formel

$$-T-Y-O-\underset{OH}{\underset{|}{\overset{O}{\overset{\|}{P}}}}-O-\underset{Z}{\underset{|}{\overset{W}{\overset{|}{CH}}}} \qquad (II)$$

bedeutet, worin T für NH oder O steht, Y Aethylen oder einen Rest der Formeln

$$\underset{(IIIa)}{Y_1-COO-Y_2} \quad \text{oder} \quad \underset{(IIIe)}{Y_1\text{-}CONH\text{-}Y_2}$$

bedeutet, worin $Y_1$ und $Y_2$ unabhängig voneinander je für unsubstituiertes Niederalkylen stehen, W Wasserstoff und Z eine 1,2-Dihydroxyäthyl- oder 2-Hydroxyäthylgruppe ist, in denen mindestens eine Hydroxygruppe mit einer gegebenenfalls ein- oder zweifach ungesättigten Alkancarbonsäure mit 16 bis 20 Kohlenstoffatomen verestert oder mit einem gegebenenfalls ein- oder zweifach ungesättigten Alkanol mit 12-18 Kohlenstoffatomen veräthert ist, oder W und Z je eine mit einer gegebenenfalls ein- oder zweifach ungesättigten Alkancarbonsäure mit 16-22 C-Atomen veresterte oder mit einem gegebenenfalls ein- oder zweifach ungesättigten Alkanol mit 12-18 C-Atomen verätherte Hydroxymethylgruppe bedeuten, sowie solche Verbindungen, worin die Bedeutungen für $A_1$ und $A_2$ vertauscht sind und ihre Salze.

Vor allem sind als Kombinationspartner Muramylpeptide der Formel I bevorzugt, worin X Carbonyl, $R_1$ Niederalkyl oder Phenyl, $R_2$, $R_4$, $R_6$ und $R_7$ Wasserstoff, $R_3$ Wasserstoff oder Methyl, $R_5$ Niederalkyl mit 1-3 C-Atomen, $A_1$ Amino und $A_2$ einen Rest der Formel

$$-NH-CH_2-CH_2-O-\underset{HO}{\overset{O}{\overset{\|}{\underset{|}{P}}}}-O-CH_2-CH(-O-R^d)-CH_2-O-R^d \qquad (IV)$$

bedeuten, worin $R^d$ für den Acylrest einer gegebenenfalls ein- oder zweifach ungesättigten Alkancarbonsäure mit 16-20 Kohlenstoffatomen steht, und ihre Salze.

Die neuen Phosphorylmuramylpeptide der Formel I können nach verschiedenen an sich bekannten Verfahren hergestellt werden. Beispielsweise werden sie hergestellt, indem man in an sich bekannter Weise eine Verbindung der Formel V,

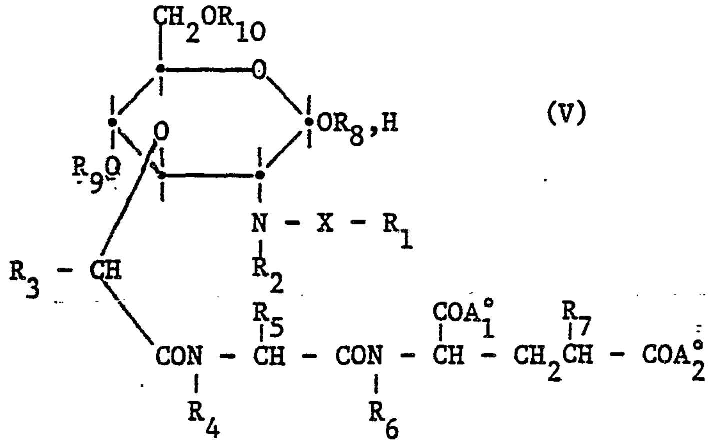

(V)

worin X, $R_1$, $R_2$, $R_3$, $R_4$, $R_5$, $R_6$ und $R_7$ die obengenannte Bedeutung haben, $R_8$, $R_9$ und $R_{10}$ Wasserstoff oder eine leicht abspaltbare Schutzgruppe darstellen und einer der Reste $A_1^\circ$ und $A_2^\circ$ eine aktivierte Hydroxygruppe darstellt und der andere veräthertes Hydroxy, Amino, Niederalkylamino oder Aminocarbonylniederalkylamino ist, mit einer Verbindung der Formel (VI),

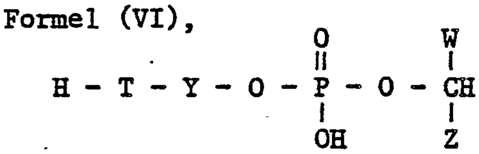

(VI)

worin T, Y, W und Z die für die Endstoffe genannte Bedeutung besitzen, kondensiert und vorhandene Schutzgruppen abspaltet.

Die aktivierte Carbonsäuregruppe $COA_1^\circ$ bzw. $COA_2^\circ$ kann beispielsweise ein Säureanhydrid z.B. mit Kohlensäureniederalkylester, wie Kohlensäureäthyl- oder -isobutylester, ein Säureazid, ein Säureamid, wie ein Imidazolid, Isoxazolid oder ein aktivierter Ester sein. Als aktivierte Ester seien insbesondere genannt: Cyanmethylester, Carboxymethylester, p-Nitrophenylester, Methoxyäthylthioester, Acetylaminoäthylthioester, p-Nitrophenylester, 2,4,5-Trichlorphenylester, N-Hydroxysuccinimidester, N-Hydroxyphthalimidester, 8-Hydroxychinolinester, N-Hydroxypiperidinester. Aktive Ester können auch gegebenenfalls mit einem Carbodiimid unter Zusatz von N-Hydroxysuccinimid oder einem unsubstituierten oder z.B. durch Halogen, Methyl oder Methoxy

substituierten 1-Hydroxybenzotriazol oder 3-Hydroxy-4-oxo-3,4-dihydrobenzo[d]-1,2,3-triazin erhalten werden.

Man bevorzugt als aktive Ester solche mit N-Hydroxysuccinimid oder dessen C-Substitutionsprodukten, wie N-Hydroxy-methyl- oder -dimethylsuccinimid, oder die Aktivierung durch Umsetzung mit einem Carbodiimid, wie Carbodiimid selbst oder 1-Aethyl-3-(3-dimethylaminopropyl)-carbodiimid.

Die dazu verwendeten Ausgangsstoffe sind bekannt oder lassen sich in an sich bekannter Weise herstellen.

Steht in den neuen Verbindungen der Formel I T für O, lässt sich diese Reaktion auch so durchführen, dass man die freie Säure mit dem Alkohol in Gegenwart eines wasserabspaltenden Mittels, wie einem Carbodiimid, z.B. Dicyclohexylcarbodiimid, und einem Amin, wie Pyridin, Dimethylaminopyridin oder einem Trialkylamin, z.B. Trimethylamin, verestert. Man kann aber auch die Carbonsäure, z.B. in Form eines Salzes, wie Natrium- oder Kaliumsalzes, mit einem reaktionsfähigen Ester des Alkohols, z.B. einem Ester mit einer starken anorganischen oder organischen Säure, wie einer Halogenwasserstoffsäure, z.B. Chlor-, Brom- oder Jodwasserstoffsäure oder einer organischen Sulfonsäure, wie p-Toluolsulfonsäure oder Methan- oder Aethansulfonsäure umsetzen. Ferner ist es auch möglich, den Alkohol gegebenenfalls als Salz, z.B. Natrium- oder Kaliumsalz, mit einer aktivierten Carbonsäure umzusetzen.

Leicht abspaltbare Schutzgruppen sind solche, die aus der Peptid- bzw. Zuckerchemie bekannt sind. Für Carboxygruppen sollen insbesondere tertiär-Butyl, Benzyl oder Benzhydryl und für Hydroxygruppen insbesondere Acylreste, z.B. Niederalkanoylreste wie Acetyl, Aroylreste, wie Benzoyl, und vor allem von der Kohlensäure sich ableitende Reste, wie Benzyloxycarbonyl oder Niederalkoxycarbonyl, oder Alkyl, insbesondere tert.-Butyl, gegebenenfalls durch Nitro, Niederalkoxy oder

Halogen substituiertes Benzyl, gegebenenfalls durch Halogen oder Niederalkoxy, wie Methoxy, substituiertes Triphenylmethyl oder Tetrahydropyranyl oder gegebenenfalls substituierte Alkylidenreste, die die Sauerstoffatome in 4- und 6-Stellung des Glucoseteils verbinden, genannt werden. Solche Alkylidenreste sind insbesondere ein Niederalkyliden-, in erster Linie der Aethyliden-, Isopropyliden- oder Propylidenrest oder auch ein gegebenenfalls substituierter, vorzugsweise in p-Stellung substituierter Benzylidenrest.

Diese Schutzgruppen können in an sich bekannter Weise abgespalten werden. So kann man sie durch saure Hydrolyse, Benzyl oder Benzylidenrest auch hydrogenolytisch, z.B. mit Wasserstoff in Gegenwart eines Edelmetall-, wie Palladium- oder Platinkatalysators entfernen.

Die folgenden Beispiele dienen zur Illustration der Erfindung. Temperaturen werden in Celsius-Graden angegeben.

Beispiel 1: Herstellung von 1000 Kapseln mit 260 mg der aktiven Ingredienzien pro Kapsel:

Zusammensetzung:

| | |
|---|---|
| Rifampicin | 250 g |
| N-Acetylmuramyl-L-alanyl-D-isoglutaminyl-L-alanyl-2-(1',2'-dipalmitoyl-sn-glycero-3'-hydroxyphosphoryloxy)-äthylamid im Gemisch mit 40-55% des Natriumsalzes | 10 g |
| Talk | 36 g |
| Weizenstärke | 24 g |
| Magnesiumstearat | 16 g |
| Laktose | 4 g |
| | 340 g |

Zubereitung:

Die pulverförmigen Substanzen werden durch ein Sieb mit einer Maschenweite von 0,6 mm geschlagen und gründlich gemischt. Mit je 340 g dieser Mischung werden mit einer Kapselfüllmaschine Gelatine-Kapseln bereitet.

Beispiel 2: Herstellung von 1000 Kapseln enthaltend 105 mg der aktiven Stoffe pro Kapsel.

Zusammensetzung:

| | |
|---|---|
| Rifampicin | 100 g |
| N-Acetyl-desmethylmuramyl-L-alanyl-D-iso-glutaminyl-2-(1',2'-dipalmitoyl-sn-glycero-3'-hydroxyphosphoryloxy)-äthylamid im Gemisch mit 40-55% des Natriumsalzes | 5 g |
| Aethyl-Cellulose | 3 g |
| Stearinsäure | 3 g |
| | 111 g |

Zubereitung:

Die Aethyl-Cellulose und die Stearinsäure werden in 120 ml Methylenchlorid gelöst, mit dem Antibiotikum versetzt, und die Masse wird durch ein Sieb von 0,6 mm Maschenweite bei einer Temperatur von ca. 40° geschlagen, wobei das Methylenchlorid verdampft. 156 g des erhaltenen Granulates werden in Gelatine-Kapseln zu 0,5 ml mit Hilfe einer Kapsel-Abfüllmaschine abgefüllt.

Beispiel 3: Herstellung eines Futtermittels enthaltend 0,005% der aktiven Stoffe.

Vor-Mischung:

| | |
|---|---|
| Rifampicin oder Chlortetracyclin | 30 g |
| N-Benzoyl-desmethylmuramyl-L-alanyl-D-iso-glutaminyl-2-(1',2'-dipalmitoyl-sn-glycero-3'-hydroxyphosphoryloxy)-äthylamid im Gemisch mit 40-55% des Natriumsalzes | 10 g |
| Staubzucker | 50 g |
| Sojabohnen Futter (mit Lösungsmitteln extrahiert) | 275 g |
| | 365 g |

Zusatzstoffe:

| | |
|---|---|
| Maismehl | 500,0 kg |
| Sojabohnenmehl, 44% Protein | 300,0 kg |
| Alfalfamehl | 13,5 kg |
| Dicalciumphosphat | 18,0 kg |
| Calciumcarbonat (gemahlen) | 4,5 kg |
| Salz | 2,3 kg |
| Fischmehl, 60% Protein | 18,0 kg |
| Stab. Fett | 27,0 kg |
| trockener Molkerückstand | 18,0 kg |
| Mangansulfat | 0,2 kg |
| Zinkoxid | 1,3 kg |
| d,l-Methionin | 0,7 kg |
| Vitamin-Vormischung | 4,5 kg |
| | 908,0 kg |

Die Vitamin-Vormischung enthält in 4,5 kg:
16 000 000 I.E. Vit. A, 1 000 000 I.E. Vit. $D_3$, 5 000 I.E. Vit. E Acetat, 6 g Vit. $K_3$, 6 mg Vit. $B_{12}$, 3 g Riboflavin, 30 g Niacin, 5 g Calcium Pantothenat und 100 g Ethoxyquin (1,2-Dihydro-6-äthoxy-2,2,4-trimethyl-chinolin) und Maismehl bis auf 4,5 kg.

Herstellungsweise:
Die Wirkstoffe und Zucker werden gründlich miteinander gemischt, durch ein Sieb von 0,6 mm Maschenweite geschlagen und dann mit dem Sojabohnenmehl vermischt. Die Vormischung wird dann in der der gewünschten Endkonzentration entsprechenden Menge zum Futtermittel zugegeben und in einem horizontalen Trommelmischer homogenisiert.

Beispiel 4: Man infiziert weisse Laboratoriums-Mäuse auf i.p. Wege mit 2 x $10^4$ cfu (colony forming units) Klebsiella pneumoniae, 0,1 x $10^4$ cfu Pasteurella multocida oder 40 x $10^4$ cfu Salmonella typhimurium in Salzlösung, so dass 90-100% der nicht behandelten

Kontrolltiere innerhalb von 48 Stunden getötet werden. Unmittelbar, sowie 3 Stunden nach der Infektion werden Gruppen zu je 10 Mäusen mit Cefroxadin, sowohl allein (ohne Muramylpeptid) als auch in Kombination mit 10 mg/kg N-Acetyl-muramyl-L-alanyl-D-isoglutaminyl-L-alanyl-2-(1',2'-dipalmitoyl-sn-glycero-3'-hydroxyphosphoryloxy)-äthylamid behandelt, wobei dieselben zweimal in Form wässriger Lösungen oder Suspensionen auf subkutanem Weg verabreicht werden. Nach 4 bzw. 10 Tagen nach erfolgter Infektion wurden die überlebenden Tiere gezählt. Die $ED_{50}$-Werte [mg/kg] für das Antibiotikum lagen bei kombinierter Gabe mit dem Muramylpeptid um ein Mehrfaches niedriger.

Beispiel 5: In analoger Weise wie in den Beispielen 1 und 2 beschrieben erhält man Kombinationspräparate, die neben den Hilfs- und Trägerstoffen die folgenden aktiven Ingredienzien (Muramylpeptide zu 40-55% in Form der Natriumsalze) in den angegebenen Mengen pro Kapsel enthalten:

a) 500 mg Cephalexin und 5 mg N-acetyl-muramyl-L-alanyl-D-isoglutaminyl-2-(1',2'-dihexadecyl-rac-glycero-3'-hydroxyphosphoryloxy)-äthylamid,
b) 750 mg Ampicillin und 40 mg N-Benzoyl-normuramyl-(α-methyl-alanyl)-D-isoglutaminyl-2-(1',2'-dioleoyl-sn-glycero-2'-hydroxyphosphoryloxy)-äthylamid,
c) 100 mg Doxycyclin und 15 mg N-Acetyl-muramyl-L-alanyl-D-glutamyl-2-(1'-hexadecyl-rac-glycero-3'-hydroxyphosphoryloxy)-äthylamid,
d) 300 mg Methacyclin und 15 mg N-Benzoyl-desmethylmuramyl-L-alanyl-D-glutamyl-γ-methylester-α-2-(1',2'-distearoyl-sn-glycero-3'-hydroxyphosphoryloxy)-äthylamid,
e) 250 mg Erythromycin-Estolat und 30 mg N-Propionyl-desmethylmuramyl-L-α-aminobutyryl-D-isoglutaminyl-γ-oxymethylcarbonyl-2-(1',3'-di[(3"R)-hydroxy-(2"S)-palmitoylamino-octadecyloxy)-2'-hydroxyphosphoryloxy]-äthylamid.

Beispiel 6: Herstellung einer sterilen Trockensubstanz zur Injektion (Lyophilisation)

500 mg Cefsulodin und 10 mg N-Acetyl-muramyl-N-methyl-L-alanyl-N,N'-dimethyl-glutaminyl-2-(1',2'-dipalmitoyl-sn-glycero-3'-hydroxyphosphoryloxy)-äthylamid im Gemisch mit 40-55% des Natriumsalzes werden unter Rühren in 5 ml Wasser gelöst. Die Lösung wird sterilfiltriert und unter aseptischen Bedingungen in ein steriles Ampullenglas (Vial) abgefüllt und lyophilisiert. Die Trockensubstanz kann nach Auflösen in Wasser oder physiologischen Lösungen für die parenterale Applikation verwendet werden.

Beispiel 7: Herstellung einer sterilen Trockensubstanz zur Injektion (Pulverabfüllung)

500 mg steriles Cefsulodin und 15 mg steriles N-Acetyl-desmethyl-muramyl-L-alanyl-D-isoglutaminyl-2-(1'-palmitoyl-2'-oleoyl-sn-glycero-3'-hydroxyphosphoryloxy)-äthylamid (Gemisch mit 40-55% des Natriumsalzes) werden unter aseptischen Bedingungen homogen gemischt und in ein Ampullenglas abgefüllt. Die Trockensubstanz kann nach Auflösen in Wasser oder physiologischen Lösungen für die parenterale Applikation verwendet werden.

Beispiel 8: In analoger Weise wie in den Beispielen 6 und 7 beschrieben erhält man sterile Trockensubstanzgemische zur Injektion, die folgende Mengen an wirksamen Bestandteilen (Muramylpeptide im Gemisch mit 40-55% ihres Natriumsalzes) enthalten:

a) 1000 mg Oxacillin (Natriumsalz) und 20 mg N-Acetyl-muramyl-L-alanyl-D-isoglutaminyl-L-alanyl-2-(3'-palmitoyl-rac-glycero-1'-hydroxyphosphoryloxy)-äthylamid,
b) 500 mg Cefazolin und 10 mg N-Acetyl-desmethylmuramyl-L-prolyl-D-glutaminyl-glycyl-2-(1',2'-dimyristoyl-sn-glycero-3'-hydroxyphosphoryloxy)-äthylamid,
c) 80 mg Gentamycin und 1 mg N-(4-Methyl-benzoyl)-desmethylmuramyl-L-valyl-D-glutaminyl-2-(1',2'-dilauroyl-sn-glycero-3'-hydroxyphosphoryloxy)-äthylamid,

d) 200 mg Doxycyclin und 0,2 mg N-Propionyl-desmethylmuramyl-L-seryl-D-isoglutaminyl-2-(1',2'-dilinoloyl-sn-glycero-3'-hydroxyphosphoryl-oxy)-äthylamid,
e) 50 mg Amphotericin B und 41 mg Natrium-Desoxycholat sowie 0,05 mg N-Acetyl-muramyl-L-lysyl-D-isoglutaminyl-2-(1',2'-distearoyl-sn-glycero-hydroxyphosphoryloxy)-äthylamid zur Herstellung der Stammlösung,
f) 500 mg Vancomycin und 0,01 mg N-4-Methoxybenzoyl-normuramyl-L-threonyl-D-isoglutaminyl-2-(1',2'-dioleoyl-sn-glycero-3'-hydroxyphosphoryloxy)-äthylamid.

Die als Ausgangsstoffe zur Herstellung der erfindungsgemässen Kombinationspräparate dienenden Muramylpeptide der Formel I werden beispielsweise analog wie in den nachfolgenden Beispielen beschrieben hergestellt.

Die Verbindungen der Formel I können weder durch einen Schmelzpunkt charakterisiert werden, noch sind spektroskopische Daten wie NMR- und IR-Spektren zur einwandfreien Charakterisierung geeignet.

Ungeeignet zur Feincharakterisierung ist ferner wegen der dominierenden Natur der Lipidteile auch die Angabe von Rf-Werten.

Da jedoch die Struktur der Ausgangsstoffe genau bekannt ist (vgl. Deutsche Offenlegungsschrift 26 55 500; die jeweils verwendete Phospholipidkomponente ist kommerziell erhältlich) und da die Verknüpfung von Phospholipid und Muramylpeptid eindeutig ist, ist damit auch die Sequenz der Bausteine im Endprodukt und dessen Struktur eindeutig festgelegt.

Beispiel 9: Zu einer Lösung von 1,4 mMol 1,2-Dipalmitoyl-sn-glycero-3-phosphoryl-äthanolamin und 2,5 mMol Triäthylamin in 25 ml eines Gemisches aus Chloroform-Methanol-Wasser, 65:25:4, wird eine Lösung

von 2 mMol N-Acetylmuramyl-L-alanyl-D-isoglutamin-N-hydroxysuccinimidester in 6,5 ml Dimethylacetamid zugetropft. Nach 18 Stunden Rühren bei 20°C wird die Lösung bei reduziertem Druck auf ca. 15 ml eingeengt; dabei entsteht eine Emulsion. Diese wird mit 200 ml Wasser verdünnt und gefriergetrocknet. Der Rückstand wird in 30 ml Wasser suspendiert und zunächst gegen Wasser, dann gegen 0,1 m Natriumphosphatpuffer pH 7 und anschliessend extensiv gegen Wasser dialysiert. Das Innendialysat, das das gewünschte Produkt und Reste von Dicyclohexylharnstoff enthält, wird mit Wasser auf 150 ml verdünnt und bei 30 000 g eine Stunde zentrifugiert. Der Ueberstand, der ein Gemisch von reinem N-Acetyl-muramyl-L-alanyl-D-isoglutaminyl-2-(1',2'-dipalmitoyl-sn-glycero-3'-phosphoryl-)äthylamid mit 40-55 Molprozent seines Natriumsalzes enthält, wird gefriergetrocknet. Die Verbindung zeigt im Dünnschichtchromatogramm auf Silicagel folgende Rf-Werte: 0,31 (in Chloroform-Methanol-Wasser, 65:25:4) bzw. 0,64 (in Chloroform-Methanol-Essigsäure-Wasser, 25:15:4:2).

Die neue Verbindung wird analytisch dadurch charakterisiert, dass die Bausteine - N-Acetylmuraminsäure, Palmitinsäure, Phosphat, L-Alanin und D-Glutaminsäure - quantitativ bestimmt werden: N-Acetylmuraminsäure wird durch mit Hilfe der Morgan-Elson-Reaktion nach der Modifikation von J.M. Ghuysen et al [in "Methods in Enzymology" 8, 629 (1966)] spektrophotometrisch bestimmt,

Phosphat wird nach Lowry et al. [J.Biol.Chem. 207, 1 (1954)] quantitativ bestimmt,

Palmitinsäure und die Aminosäuren werden in einem Totalhydrolysat (6N HCl, 24 Std. 110°C) gaschromatographisch bzw. mit Hilfe eines Aminosäureanalysators unter Verwendung von Pentadecansäure bzw. Norleucia als interne Standards quantitativ bestimmt.

Die gefundenen molaren Verhältnisse, bezogen auf Phosphat, sind wie folgt:

$PO_4'''$: N-Acetylmuraminsäure: L-Alanin:D-Glutaminsäure:Palmitinsäure = 1:0,92:0,91:0,95:2,18.

Der N-Acetylmuramyl-L-alanyl-D-isoglutamin-N-hydroxy-succinimidester, der als Ausgangsstoff verwendet wird, lässt sich z.B. wie folgt herstellen:

2 mMol N-Acetylmuramyl-L-alanyl-D-isoglutamin, 2,2 mMol N-Hydroxysuccinimid und 2,2 mMol Dicyclohexylcarbodiimid werden in 6,5 ml Dimethylacetamid gelöst und 18 Stunden bei 20°C gerührt. Der ausgefallene Dicyclohexylharnstoff wird abgetrennt und die Lösung direkt für die Kondensation mit dem Phospholipid verwendet.

Das 1,2-Dipalmitoyl-sn-glycero-3-phosphoryl-äthanolamin, das als Ausgangsstoff verwendet wird, ist ein kommerziell erhältliches synthetisches Präparat.

Beispiel 10: In analoger Weise zu Beispiel 1 erhält man N-Acetylmuramyl-L-alanyl-D-isoglutaminyl-L-alanyl-2-(1',2'-dipalmitoyl-sn-glycero-3'-phosphoryl-)äthylamid, ausgehend von 1,2-Dipalmitoyl-sn-glycero-3-phosphoryl-äthanolamin und N-Acetylmuramyl-L-alanyl-D-isoglutaminyl-L-alanin-N-hydroxysuccinimidester. Rf-Wert im Dünnschichtchromatogramm auf Silicagel: 0,3 (im System Chloroform-Methanol-Wasser, 65:25:4).

Patentansprüche

1. Pharmazeutische Präparate, die mindestens ein Antibiotikum und mindestens ein Muramylpeptid der Formel (I) und/oder ein Salz desselben enthalten,

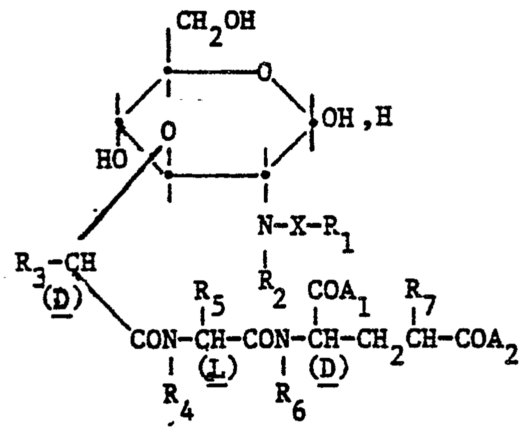

(I)

worin X Carbonyl oder Carbonyloxy, $R_1$ gegebenenfalls substituiertes Alkyl oder Aryl, $R_2$, $R_4$ und $R_6$ Wasserstoff oder Niederalkyl, $R_3$ Wasserstoff oder Niederalkyl, $R_5$ Wasserstoff, Niederalkyl, freies oder funktionell abgewandeltes Hydroxyniederalkyl, freies oder funktionell abgewandeltes Mercaptoniederalkyl, gegebenenfalls substituiertes Aminoniederalkyl, Cycloalkyl, Cycloalkylniederalkyl, gegebenenfalls substituiertes Aryl oder Aralkyl, stickstoffhaltiges Heterocyclyl oder Heterocyclylniederalkyl, oder $R_4$ und $R_5$ zusammen auch Alkylen mit 3-4 Kohlenstoffatomen, $R_7$ Wasserstoff oder gegebenenfalls verestertes oder amidiertes Carboxyl und einer der Reste $A_1$ und $A_2$ einen Rest der Formel

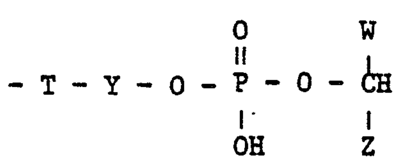

(II)

bedeutet, worin T für NH oder O steht, Y eine gegebenenfalls substituierte Alkylengruppe, die auch durch ein oder zwei Oxycarbonyl und/oder Iminocarbonyl unterbrochen sein kann, W Wasserstoff und Z

eine 1,2-Dihydroxyäthyl- oder 2-Hydroxyäthylgruppe darstellen, in denen mindestens eine Hydroxygruppe mit einer gegebenenfalls ungesättigten, langkettigen aliphatischen Carbonsäure verestert oder mit einem gegebenenfalls ungesättigten, langkettigen aliphatischen Alkohol veräthert ist, oder W und Z je eine mit einer gegebenenfalls ungesättigten langkettigen aliphatischen Carbonsäure veresterte oder mit einem gegebenenfalls ungesättigten langkettigen aliphatischen Alkohol verätherte Hydroxymethylgruppe darstellen, und der andere der Reste $A_1$ und $A_2$ freies oder veräthertes Hydroxy, freies oder alkyliertes Amino, Niederalkylamino oder Aminocarbonylniederalkylamino ist und ihre Salze.

2. Präparate nach Anspruch 1, die mindestens ein Antibiotikum aus der Gruppe der β-Lactamantibiotika, Aminoglycoside, Tetracycline, Macrolide, Lincomycine, Polyenantibiotika, Polypeptidantibiotika, Anthracycline, Chlor- oder Thiamphenicole,Cycloserine, Fusidinsäuren oder Rifamycine enthalten.

3. Präparate nach Anspruch 1, die als Antibiotikum ein Penicillin, Cephalosporin, Penem, Nocardicin, Thienamycin, eine Clavulanverbindung, ein Streptomycin, Neomycin, Tobramycine, ein Kanamycin, Gentamycin oder Sisomycin enthalten.

4. Präparate nach Anspruch 1, die als Antibiotikum Amoxycillin, Ampicillin, Carbenicillin, Cloxacillin, Cyclacillin, Dicloxacillin, Mecillinam, Methicillin, Penicillin G, Penicillin V, Pivampicillin, Sulbenicillin, Azlocillin, Ticarcillin, Mezlocillin, Pivmecillinam, 6-(4-endo-Azatricyclo[$5.2.2.0^{2,6}$]undec-8-enyl)-methylenamino-penicillansäure, Cefaclor, Cefazaflur, Cefazolin, Cefadroxil, Cefoxitin, Cefuroxim, Cephacetril, Cephalexin, Cephaloglycin, Cephaloridine, Cephalotin, Cefamandol, Cephanon, Cephapirin, Cefatrizin, Cephardin, Cefroxadin {7β-[D-2-amino-2-(1,4-cyclohexadienyl)-acetamido]-3-methoxy-3-cephem-4-carbonsäure}, Cefsulodin, Cefotaxim, Cefotiam,

Ceftezol, Cefazedon, Nocardicin A, Thienamycin, Clavulansäure, Streptomycin, Streptomycin A, Neomycin B, Tobramycin, Dibekacin, Gemische von Kanamycin A, B und C, Amicacine, Gemische von Gentamycin A, $C_1$, $C_2$ oder $C_{1a}$, Sisomycin, Netilmicin, Lividomycin, Ribocamycin, Paromomycin, Tetracyclin, Doxycyclin, Chlortetracyclin, Oxytetracyclin, Methacyclin, Maridomycin, Spiramycine, Erythromycine, Oleandomycine, Lincomycine, Amphothericin B und dessen Methylester, Nystalin, Colistin, Gramicidin S, Polymyxin B, Virginamycin, Thyrothricin, Viomycin, Vancomycin, Rifamycin S, -SV, B oder deren halbsynthetische Derivate enthalten.

5. Präparate nach Anspruch 1, die als Antibiotikum Cephacetril, Cefadroxil, Rifampicin, Cefsulodin, Cefroxadin, Bicozamycin oder Cefotiam enthalten.

6. Präparate nach einem der Ansprüche 1-5, die zwischen 50 und 1000 mg Antibiotikum und bei oral verabreichbaren Präparaten zwischen 1 mg und 50 mg Muramylpeptid bzw. bei oral verabreichbaren magensaftbeständigen Manteltabletten oder bei injizierbaren Präparaten zwischen 0,01 mg und 50 mg Muramylpeptid pro Dosiseinheit zusammen mit einem pharmazeutisch verwendbaren Trägermaterial enthalten.

7. Präparate nach einem der Ansprüche 1-6, die ein Muramylpeptid der Formel I gemäss Anspruch 1 und/oder ein Salz desselben enthalten, worin X Carbonyl, $R_1$ gegebenenfalls substituiertes Alkyl mit bis zu 18 C-Atomen oder Aryl mit bis zu 30 C-Atomen, $R_2$, $R_3$, $R_4$ und $R_6$ Wasserstoff oder Niederalkyl, $R_5$ Wasserstoff, gegebenenfalls durch Hydroxy, Niederalkoxy, Mercapto, Niederalkylmercapto, Amino, Niederalkylamino oder Halogen substituiertes Niederalkyl, Cycloalkyl oder Cycloalkylniederalkyl, worin der Cycloalkylrest 4-6 Kohlenstoffatome enthält, gegebenenfalls substituiertes Phenyl oder Phenylniederalkyl,

ein oder zwei Azaatome enthaltendes Heterocyclyl oder Heterocyclylniederalkyl mit jeweils 5-6 Ringgliedern, oder $R_4$ und $R_5$ zusammen auch Alkylen mit 3-4 Kohlenstoffatomen, $R_7$ Wasserstoff und einer der Reste $A_1$ und $A_2$ einen Rest der Formel

$$-T-Y-O-\underset{\displaystyle OH}{\underset{|}{\overset{\displaystyle O}{\overset{\|}{P}}}}-O-\underset{\displaystyle Z}{\underset{|}{\overset{\displaystyle W}{\overset{|}{CH}}}} \qquad (II)$$

bedeuten, worin T für HN oder O steht, Y gegebenenfalls substituiertes Alkylen mit bis zu 20 C-Atomen, das auch durch Carbonyloxy oder Carbonylimino unterbrochen sein kann, W Wasserstoff und Z eine 1,2-Dihydroxyäthyl- oder 2-Hydroxyäthylgruppe darstellen, in denen mindestens eine Hydroxygruppe mit einer gegebenenfalls ungesättigten langkettigen aliphatischen Carbonsäure mit bis zu 30 C-Atomen oder mit einer Mycolsäure verestert oder mit einem gegebenenfalls ungesättigten langkettigen aliphatischen Alkohol mit bis zu 30 C-Atomen veräthert ist, oder W und Z je eine mit einer gegebenenfalls ungesättigten, langkettigen aliphatischen Carbonsäure mit bis zu 30 C-Atomen oder mit einer Mycolsäure veresterte oder mit einem gegebenenfalls ungesättigten, langkettigen aliphatischen Alkohol mit bis zu 30 C-Atomen verätherte Hydroxymethylgruppe darstellen, und der andere der Reste $A_1$ und $A_2$ freies oder veräthertes Hydroxy, Amino, Niederalkylamino oder Aminocarbonylniederalkylamino ist.

8. Präparate gemäss einem der Ansprüche 1 bis 7, die ein Muramylpeptid der Formel I gemäss Anspruch 1 und/oder ein Salz desselben enthalten, worin X Carbonyl, $R_1$ gegebenenfalls durch Hydroxy, Niederalkoxy oder Halogen substituiertes Niederalkyl oder gegebenenfalls durch Hydroxy, Niederalkoxy, Niederalkyl oder Halogen substituiertes Phenyl, $R_2$, $R_4$ und $R_6$ Wasserstoff oder Methyl, $R_3$ Wasserstoff, Methyl oder Aethyl, $R_5$ Wasserstoff, gegebenenfalls durch Hydroxy, Niederalkoxy, Mercapto, Niederalkylmercapto oder Halogen substituiertes Niederalkyl mit 1-7 Kohlenstoffatomen, 4-Amino-butyl, Cycloalkyl oder Cycloalkylniederalkyl, worin der Cycloalkylrest 4-6 Kohlenstoffatome und der Niederalkylrest 1-3 Kohlenstoffatome enthält, gegebenenfalls durch Hydroxy, Niederalkoxy oder Halogen substituiertes Phenyl oder Phenylniederalkyl mit 1-3 Kohlenstoffatomen im Niederalkylrest, 4-Imidazolyl-methyl, 3-Indolyl-methyl oder $R_4$ und $R_5$ zusammen auch Alkylen mit 3-4 Kohlenstoffatomen, $R_7$ Wasserstoff und einer der Reste $A_1$ und $A_2$ einen Rest der Formel

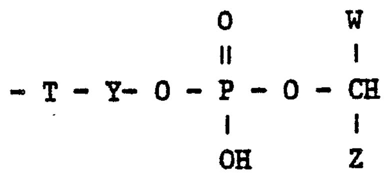

(II)

bedeutet, worin T für HN oder O steht, Y gegebenenfalls substituiertes Niederalkylen oder einen Rest der Formeln

$$-Y_1 - COO - Y_2 \quad \text{(IIIa)}$$

oder

$$-Y_1 - CONH - Y_2 \quad \text{(IIIe)}$$

bedeutet, worin $Y_1$ und $Y_2$ unabhängig voneinander je für Niederalkylen mit 1-7 Kohlenstoffatomen stehen, das gegebenenfalls durch Hydroxy, Niederalkoxy, Mercapto, Methylthio, Phenyl, 4-Imidazolyl oder 3-Indolyl substituiert ist, W Wasserstoff, und Z eine 1,2-Dihydroxyäthyl- oder 2-Hydroxyäthylgruppe darstellen, in denen mindestens eine Hydroxygruppe mit einer gegebenenfalls ein- oder zweifach ungesättigten aliphatischen Carbonsäure mit 14-24 Kohlenstoffatomen oder mit einer natürlichen oder synthetischen Mycolsäure verestert oder mit einem gegebenenfalls ein- oder zweifach ungesättigten aliphatischen Alkohol mit 10-20 Kohlenstoffatomen veräthert ist, oder W und Z je eine mit einer gegebenenfalls ein- oder zweifach ungesättigten aliphatischen Carbonsäure mit 16-22 C-Atomen oder mit einer natürlichen oder synthetischen Mycolsäure veresterte oder mit einem gegebenenfalls ein- oder zweifach ungesättigten aliphatischen Alkohol mit 12-18 C-Atomen verätherte Hydroxymethylgruppe bedeuten, und der andere der Reste $A_1$ und $A_2$ Hydroxy, Niederalkoxy, Amino, Niederalkylamino oder Aminocarbonylniederalkylamino ist.

9. Präparate gemäss einem der Ansprüche 1-8, die ein Muramylpeptid der Formel I gemäss Anspruch 1 und/oder ein Salz desselben enthalten, worin X Carbonyl, $R_1$ Niederalkyl oder Phenyl, $R_2$, $R_6$ und $R_7$ Wasserstoff, $R_3$ und $R_4$ Wasserstoff oder Methyl, $R_5$ Wasserstoff, gegebenenfalls durch Phenyl substituiertes Niederalkyl mit 1-7 C-Atomen oder $R_4$ und $R_5$ zusammen auch Trimethylen bedeuten, worin $A_1$ für Hydroxy, Niederalkoxy, Amino, Niederalkylamino oder Aminocarbonylniederalkylamino steht und $A_2$ einen Rest der Formel

$$- T - Y - O - \underset{\underset{OH}{|}}{\overset{\overset{O}{\|}}{P}} - O - \underset{\underset{Z}{|}}{\overset{\overset{W}{|}}{CH}} \qquad (II)$$

bedeutet, worin T für NH oder O steht, Y Aethylen oder einen Rest der Formeln

$$\underset{(IIIa)}{Y_1 - COO - Y_2} \qquad \text{oder} \qquad \underset{(IIIe)}{Y_1\text{-}CONH\text{-}Y_2}$$

bedeutet, worin $Y_1$ und $Y_2$ unabhängig voneinander je für unsubstituiertes Niederalkylen stehen, W Wasserstoff und Z eine 1,2-Dihydroxyäthyl- oder 2-Hydroxyäthylgruppe ist, in denen mindestens eine Hydroxygruppe mit einer gegebenenfalls ein- oder zweifach ungesättigten Alkancarbonsäure mit 16 bis 20 Kohlenstoffatomen verestert oder mit einem gegebenenfalls ein- oder zweifach ungesättigten Alkanol mit 12-18 Kohlenstoffatomen veräthert ist, oder W und Z je eine mit einer gegebenenfalls ein- oder zweifach ungesättigten Alkancarbonsäure mit 16-22 C-Atomen veresterte oder mit einem gegebenenfalls ein- oder zweifach ungesättigten Alkanol mit 12-18 C-Atomen verätherte Hydroxymethylgruppe bedeuten, sowie solche Verbindungen, worin die Bedeutungen für $A_1$ und $A_2$ vertauscht sind.

10. Präparate gemäss einem der Ansprüche 1-9, die ein Muramylpeptid der Formel I gemäss Anspruch 1 und/oder ein Salz desselben enthalten, worin X Carbonyl, $R_1$ Niederalkyl oder Phenyl, $R_2$, $R_4$, $R_6$ und $R_7$ Wasserstoff, $R_3$ Wasserstoff oder Methyl, $R_5$ Niederalkyl mit 1-3 C-Atomen, $A_1$ Amino und $A_2$ einen Rest der Formel

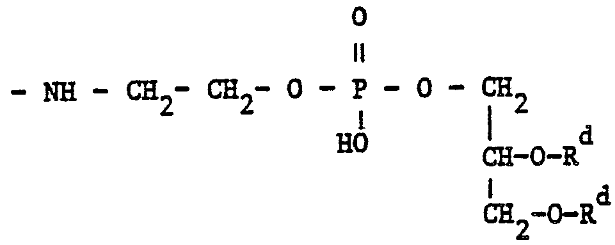

(IV)

bedeuten, worin $R^d$ für den Acylrest einer gegebenenfalls ein- oder zweifach ungesättigten Alkancarbonsäure mit 16-20 Kohlenstoffatomen steht.

11. Verfahren zur Steigerung der antibiotischen Aktivität von Antibiotika, dadurch gekennzeichnet, dass man mindestens ein Antibiotikum gemäss einem der Ansprüche 2-6 mit mindestens einem Muramylpeptid gemäss einem der Ansprüche 1 und 6-10 zusammen verabreicht.

12. Futtermittel und Futtermittelzusätze, die mindestens ein Antibiotikum gemäss einem der Ansprüche 2-5 und mindestens ein Muramylpeptid gemäss einem der Ansprüche 7-10 enthalten.

**0056560**

Nummer der Anmeldung

EP 81 81 0013

Europäisches Patentamt

## EUROPÄISCHER TEILRECHERCHENBERICHT,

der nach Regel 45 des Europäischen Patentübereinkommens für das weitere Verfahren als europäischer Recherchenbericht gilt

### EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| E | EP - A - 0 027 258 (CIBA-GEIGY AG)<br>* Seite 67, Anspruch 25; Seite 93, Anspruch 27 *<br>-- | 1-10, 12 |
| E | EP - A - 0 025 495 (CIBA-GEIGY AG)<br>* Seite 14, Abschnitt 2 *<br>-- | 1-10, 12 |
| T | EP - A - 0 026 746 (CIBA-GEIGY AG)<br>* Seiten 17-21; Ansprüche 1-12 *<br>-- | 1-10, 12 |
| | DE - A - 2 655 500 (CIBA-GEIGY AG)<br>* Seite 28, Zeilen 12-15 *<br>-- | 1-10, 12 |
| | FR - A - 2 387 037 (KUREHA KAGAKU KOGYO K.K.)<br>* Seite 1, Zeile 26 - Seite 2, Zeile 2 *<br>& DE - A - 2 816 087<br>-- ./. | 1-10, 12 |

KLASSIFIKATION DER ANMELDUNG (Int Cl.³)

A 61 K 45/06
37/02
31/70
31/71
A 23 K 11/16
11/17//
(A 61 K 37/02
31/71)
(A 61 K 37/02
31/43)
(A 61 K 37/02
31/65)
(A 61 K 31/71
31/70)

RECHERCHIERTE SACHGEBIETE (Int Cl.³)

A 61 K 37/02
31/70
31/71
C 07 H 15/00
A 23 K 1/16
1/17

### UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-10,12

Unvollständig recherchierte Patentansprüche:

Nicht recherchierte Patentansprüche: 11: Verfahren zur chirurgischen oder

Grund für die Beschränkung der Recherche:

therapeutischen Behandlung des menschlichen oder tierischen Körpers. (Siehe Art. 52(4) des Europäischen Patentübereinkommens).

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 23-09-1981 | BRINKMANN |

0056560

Nummer der Anmeldung

Europäisches Patentamt

# EUROPÄISCHER TEILRECHERCHENBERICHT

EP 81 81 0013

-2-

| EINSCHLÄGIGE DOKUMENTE | | | KLASSIFIKATION DER ANMELDUNG (Int. Cl.) |
|---|---|---|---|
| Kategorie | Kennzeichnung des Dokuments mit Angabe, soweit erforderlich der maßgeblichen Teile | betrifft Anspruch | |
| | DE - A - 2 331 144 (ANVAR)<br>* Seite 5, Zeile 27 - Seite 6, Zeile 3 *<br>---- | 1-10, 12 | |
| | | | RECHERCHIERTE SACHGEBIETE (Int. Cl.³) |